# EUROPEAN PATENT APPLICATION

(11) **EP 0 816 510 A1**
(43) Date of publication of application: **07.01.1998**
(21) Application number: 96942608.9
(22) Date of filing: 20.12.1996
(51) Int. Cl.: C12P 21/02, C12N 15/62, C12N 15/24

(54) **PROCESS FOR PRODUCING BIOLOGICALLY ACTIVE FUSED PROTEINS**

(30) Priority: 22.12.1995 JP 334875/95; 22.12.1995 JP 350364/95
(71) Applicant: TORAY INDUSTRIES, INC., Tokyo 103 (JP); TORAY RESEARCH CENTER, INC., Chuo-ku, Tokyo 103 (JP)
(72) Inventor: NAKATANI, Izumi, Kanagawa 241 (JP); TOMURA, Michio, Toyonaka-shi-Osaka 560 (JP); KANAI, Shozo, Kanagawa 247 (JP); FUJIWARA, Hiromi, Kobe-shi,Hyogo 658 (JP); AKIYAMA, Hideo, Kanagawa 248 (JP)
(74) Representative: Kador & Partner
(86) International application number: JP9603742
(87) International publication number: WO9723639

(57) **Abstract**

A process for producing biologically active fused proteins whereby novel biologically active fused proteins usable as a remedy or a diagnostic drug or applicable to gene therapy can be efficiently produced by appropriately selecting the optimum linker polypeptide. This process comprises incubating transformants obtained by the transformation by expression vectors which contain the DNA sequences encoding the amino acid sequences of proteins represented by the following formula: A-X-B wherein A and B represent respectively the subunits of a dimer protein or biologically active proteins and may be the same; and X represents a linker polypeptide.

## Description

### TECHNICAL FIELD

The present invent ion relates to a method for producing a dimer protein exhibiting a biological activity or a bioactive fusion protein composed of two kinds of proteins. In particular, the present invention relates to a method for producing a bioactive fusion protein utilizing a linker polypeptide.

### BACKGROUND ART

Proteins play important parts in supporting life, and utilization of functions of proteins is a great challenge. Thanks to development of recombinant DNA techniques, a great number of useful foreign proteins have been expressed in prokaryotic cells or eukaryotic cells or insects, such as silk worms, transformed by expression vectors containing DNA sequences encoding those foreign proteins. However, many problems have arisen accompanying such technologies, for example, problems regarding production of dimer proteins having disulfide bonds, or production of membrane proteins, and the like.

For example, to produce a dimer protein composed of two subunit proteins, represented by interleukin-12 (IL12) known as a natural killer cell simulating factor, there is a requirement that the two subunit proteins be simultaneously expressed in equal amounts. However, methods using existing expression vectors have problems of, for example, non-uniformity in the amounts of subunit proteins expressed, extremely low transformation efficiencies, incapability of using insect expression systems employing silk worms or the like.

A generally known method for producing a dimer protein is a co-transfection method wherein transformants are obtained through a mixture of two expression vectors containing genes encoding different subunit proteins. However, this method also has many problems to be solved. For example, the method produces not only transformants containing the two types of expression vectors, but also transformants containing only one type of expression vectors are also produced. Moreover, the amount of each subunit protein expressed in the transformants containing the two types of expression vectors varies.

To overcome such problems, fusion protein production methods using linker peptides (polypeptide) have recently been developed (for example, WO09204455, WO 921568, EP 610046, WO 9319777, WO 9215682, WO 9102754, EP 467839, and EP 281418). However, no finding has been obtained regarding production of a dimer protein with a disulfide bond, or production of a mature protein utilizing intracellular processing, or the like.

Interleukin-12 is briefly described here. Interleukin-12 is a protein known as a natural killer cell stimulating factor (NKSF) (Kobayashi, M. et al. J. Exp. Med. 170, 827 (1989)), or a cytotoxic lymphocyte maturation factor (CLMF) (Gately, M.K., J. Immunol., 136, 1274 (1986)). It is also known to act to increase the production of interferon γ from peripheral blood mononuclear cells (Stem, A.S. et al., Proc. Natl. Acad. Sci. USA, 87, 6808(1990)) and to induce THL cells, a subtype of helper T cells (Th) (Manetti, R. et al., J. Exp. Med., 177, 1199(1993)). It has been revealed that interleukin-12 is composed of two types of subunits, that is, a 35KDa subunit and a 40KDa subunit (Podlasky, F.J. et al., Arch. Biochem. Biophys. 294, 230 (1991)).

In a production method for interleukin-12, genes encoding the two interleukin-12-constituting subunits of gp 35 and gp 40 are separately inserted into existing expression vectors, and then transformants are obtained through mixture of the vectors (Japanese Patent Laid-Open No. Hei 5-294999). This method produces not only transformants containing the two types of vectors but also transformants containing only one type of vectors. Moreover, the amounts expressed in the transformants containing the two types of vectors according to this method vary. Therefore, interleukin-12, which is a hetero-dimer, cannot be efficiently produced by this method.

Production of interleukin-12, a heterodimer, is difficult as described above. Unlike the case of homodimers, the two subunits of the heterodimer must be expressed simultaneously in equal amounts to produce a bioactive protein. Therefore, it becomes necessary to incorporate a plurality of promoters into an existing expression vector, thereby creating possibilities of great variation in expressed amount of each unit or significantly reduced transformation efficiencies.

### DISCLOSURE OF THE INVENTION

It is an object of the present invention to provide an excellent method of producing a bioactive fusion protein using a linker polypeptide and to provide a method regarding selection of an optimal linker polypeptide.

Through careful researches, the present inventors have found that the above object can be achieved by a method of producing a bioactive fusion protein characterized by culturing a transformant obtained throughy transformation using an expression vector containing a DNA sequence encoding an amino acid sequence of a bioactive protein represented by the following formula:

A-X-B

(wherein A and B represent respectively subunits of a dimer protein or bioactive monomers in their native form where A and B may represent the same, and X represents a linker polypeptide), thereby accomplishing the present invention.

In the present invention, it is preferable that X be:
(1) a linker polypeptide located between a C terminal of the protein A and an N terminal of the protein B, the second amino acid residue from the last at the C terminal of the linker polypeptide and the last amino acid residue at the C terminal being Lys-Arg and the first and second amino acid residues at the N terminal of the linker polypeptide being Arg-Arg, and the linker polypeptide being cut from the fusion protein by intracellular processing in a host; or
(2) a linker polypeptide located between a C terminal of the protein and an N terminal of the protein B, the second amino acid residue from the last at the C terminal of the linker polypeptide and the last amino acid residue at the C terminal and the first and second amino acid residues at the N terminal of the linker polypeptide being constituted by basic amino acids selected from a group consisting of Arg and Lys, or hydrophobic amino acids selected from a group consisting of Pro, Phe, Leu, Tyr, Ile, Leu and Val.

The present invention also relates to an expression vector characterized by containing a DNA sequence encoding the above amino acid sequence and to a transformant obtained by transforming a host cell with the expression vector.

Furthermore, in the present invention, it is preferable that A and B be either the 40 KDa subunit or the 35 KDa sub-unit of interleukin-12, and that the synthesis object substance be interleukin-12.

### BRIEF DESCRIPTION OF THE DRAWINGS

Fig. 1 schematically illustrates a process of preparing the subunits gp35, gp40 of IL12 by PCR and producing a plasmid pUC18 having a gp40-gp35 gene structure.
Fig. 2 shows a nucleic acid encoding InsC region in gp40-(InsCI-N)-gp35 and flanking IL12 pg35 and IL12 gp40 sequences as well as the encoded amino acid sequence.
Fig. 3 shows a nucleic acid encoding InsC region in gp40-(InsCII-N)-gp35 and flanking IL12 pg35 and IL12 gp40 sequences as well as the encoded amino acid sequence.
Fig. 4 shows a nucleic acid encoding InsC region in gp40-(InsCI-C)-gp35 and flanking IL12 pg35 and IL12 gp40 sequences as well as the encoded amino acid sequence.
Fig. 5 shows a nucleic acid encoding InsC region in gp40-(InsCII-C)-gp35 and flanking IL12 pg35 and IL12 gp40 sequences as well as the encoded amino acid sequence.
Fig. 6 illustrates a process of constructing a gp40-(InsCI-N)-gp35 fusion protein expression vector InsCI-N/pSVL (InsCI=insulin type I C chain).
Fig. 7 illustrates a process of constructing a gp40-(InsCI-C)-gp35 fusion protein expression vector InsCI-C/pSVL (InsCI=insulin type I C chain).
Fig. 8 illustrates a process of constructing a gp40-(InsCII-N)-gp35 fusion protein expression vector InsCII-N/pSVL (InsCII=insulin type II C chain).
Fig. 9 illustrates a process of constructing a gp40-(InsCII-C)-gp35 fusion protein expression vector InsCII-C/pSVL (InsCII=insulin type II C chain).
Fig. 10 shows a a nucleic acid encoding InsC region in gp40-(E. coli-N)-gp35 and flanking IL12 pg35 and IL12 gp40 sequences as well as the encoded amino acid sequence.
Fig. 11 illustrates a process of constructing a gp40-(E. coli-N)-gp35 fusion protein expression vector E. coli-N/pSVL (E.coli-N=Escherichia coil-derived DNA fragment).
Fig. 12 shows results of a quantitative assay of a production of IL-12 wherein the supernatants of the filtered and sterilized COS cells were serially diluted.
Fig. 13 shows results of immunoblotting with an anti-gp40 monoclonal antibody preceded by SDS-polyacrylamide electrophoresis of 10-fold concentrations of the supernatants of the filtered and sterilized COS cells on a 10% polyacrylamide gel under non-reducing conditions.
Fig. 14 shows results of immunoblotting with an anti-gp40 monoclonal antibody preceded by SDS-polyacrylamide electrophoresis of 10-fold concentrations of the supernatants of the filtered and sterilized COS cells on a 10% polyacrylamide gel under reducing conditions.
Fig. 15 schematically illustrates a process of preparing an IL2 gene by PCR and producing a plasmid pUC18 having IL2+S and IL2-S gene structures.
Fig. 16 shows a nucleic acid sequence of an InsC region of IL2-(InsC I-N)-IL2 and flanking IL2 sequences , as well as the encoded amino acid sequence.
Fig. 17 shows a nucleic acid sequence of an InsC region of IL2-(InsC II-N)-IL2 and flanking IL2 sequences , as well as the encoded amino acid sequence.
Fig. 18 shows a nucleic acid sequence of an InsC region of IL2-(InsC I-C)-IL2 and flanking IL2 sequences , as well as the encoded amino acid sequence.
Fig. 19 shows anucleic acid sequence of an InsC region of IL2-(InsC II-C)-IL2 and flanking IL2 sequences , as well as the encoded amino acid sequence.
Fig. 20 illustrates a process of constructing an IL2-(InsC I-N)-IL2 fusion protein expression vector IL2/InsCI-N/pSVL (InsCI=insulin type I C chain).
Fig. 21 illustrates a process of constructing an IL2-(InsC I-C)-IL2 fusion protein expression vector IL2/InsCI-C/pSRα(InsCI=insulin type I C chain).
Fig. 22 illustrates a process of constructing an IL2-(InsC II-N)-IL2 fusion protein expression vector IL2/InsCII-N/pSRα(InsCII=insulin type II C chain).
Fig. 23 illustrates a process of constructing an IL2-(InsC II-C)-IL2 fusion protein expression vector IL2/InsCII-C/pSRα(InsCII=insulin type II C chain).
Fig. 24 shows results of a quantitative assay of bioactivity of IL-2 wherein the supernatants of the filtered and sterilized COS cells were serially diluted.
Fig. 25 shows results of SDS-polyacrylamide electrophoresis of 10-fold concentrations of the supernatants of the filtered and sterilized COS cells on a 4-20% gradient gel under non-reducing conditions.
Fig. 26 shows results of SDS-polyacrylamide electrophoresis of 10-fold concentrations of the supernatants of the filtered and sterilized COS cells on a 4-20% gradient gel under reducing conditions (0.1 uL of 14.4M β-mercaptoethanol was added to a total volume of 20 µL).
Fig. 27 illustrates a process of constructing a gp40-(InsC I-N)-gp35 fusion protein expression vector InsCI-N/pSRα (InsCI=insulin type I C chain).
Fig. 28 illustrates a process of constructing a gp40-(InsC I-C)-gp35 fusion protein expression vector InsCI-C/pSRα (InsCI=insulin type I C chain).
Fig. 29 illustrates a process of constructing a gp40-(InsC II-N)-gp35 fusion protein expression vector InsCII-N/pSRα(InsCII=insulin type II C chain).
Fig. 30 illustrates a process of constructing a gp40-(InsC II-C)-gp35 fusion protein expression vector InsCII-C/pSRα(InsCII=insulin type II C chain).
Fig. 31 illustrates a process of constructing a gp40-(E. coli-N)-gp35 fusion protein expression vector E. coli-N/pSRα(E. coli-N=Escherichia coil-derived DNA fragment).
Fig. 32 shows results calculation and comparison of IL12 concentrations in the supernatants of the filtered and sterilized COS cells, based on the growth curve obtained by a 2D6 assay wherein the supernatants were serially diluted.

### BEST MODES FOR CARRYING OUT THE INVENTION

According to the present invention, in the protein represented by the following formula:

A-X-B

(wherein A and B represent respectively subunits of a dimer protein or bioactive monomers in their native form, where A and B may represent the same, and X represents a linker polypeptide)
the bioactive protein used refers to any protein being capable of changing a control function in an organism and originating from prokaryotes or eukaryotes, particularly, higher eukaryotes, for example, mammals.

Cited as examples of such protein are: hormones such as cerectin, chymosin, calcitonin, luteinizing hormone, parathyroid hormone, adrenocorticotropic hormone, melanocyte stimulating hormone, β-lipotropin, urogasterone, insulin or the like, epidermal growth factor (EGF), insulin-like growth factors represented by IGF-1 or IGF-2, and their binding proteins (IGPBP), growth factors such as mouse cell growth factor, nerve growth factor, glia-derived nerve cell growth factor, platelet-derived growth factor (PDGP), epithelial growth factor, transforming growth factor (TGF), hematopoietic stem cell growth factor or the like, growth hormones such as human or bovine growth hormone or the like, interleukins represented by interleukin-2, interleukin 8, interleukin-12 or the like, blood granulocytic cell colony stimulating factors, human macrophage migration inhibiting factors (MIF), interferons such as interferon α, β or γ or the like, osteogenesis factors, α1-anti-trypsin, protease inhibitors such as SLPI or the like, hepatitis virus antigens such as B-type hepatitis virus surface or core antigens, A-type hepatitis virus antigens, C-type hepatitis virus antigens or the like, plasminogen activators such as tissue plasminogen activators, urokinase, hybrid plasminogen activators or the like, blood coagulation factors represented by tick anticoagulant peptides (TAP), tumor necrosis factor, somatostatin, renin, human calcitonin-associated peptides, and blood coagulation factors IX and VIIIc, erythropoietin, egrins such as egrin C, disulfate hirudin, corticostatin, extatin, cystatin, human superoxide dismutase, human tyrosine kinase, virus thymidine kinase, β-lactamase, glucose isomerase or the like. Further cited are, for example, cytokine receptors represented by human interleukin-2 receptors, human interleukin-6 receptors, erythropoietin receptors or the like, interferon receptors, blood granulocytic cell colony stimulating factor receptors, epidermal growth factor receptors, hematopoietic stem cell growth factor receptors, an immunoglobulin super gene family including CD2, CD3, CD4 and the like, T cell antigen receptors, immunoglobulins such as immunoglobulin D, E, G or the like, human-mouse hybrid immunoglobulins, immunoglobulin binding factors represented by immunoglobulin E binding factor, GTP binding proteins such as low-molecular GTP binding proteins, GTP binding protein γ sub-unit protein or the like, estrogen receptors, andorogen receptors, glucocortinoid receptors, vitamin receptors represented by vitamin D3 receptors, parathyroid hormone, and the like. Preferred examples are peptidases such as γ-glutamyl transpeptidase, interferons, particularly, interferon γ, immunoglobulins, particularly, the Fc chain or Fab chain of immunoglobulin G, interleukins represented by interleukin-1, -2, -6, or -12 and their receptors or soluble receptors, cytokine common receptors represented by gp130 or interleukin-2β-subunit receptor, human tissue plasminogen activators, transforming growth factor TGF β or its receptor, GTP binding proteins such as GTP binding protein γ sub-unit protein, and the like.

According to the present invention, X in the formula A-X-B is preferably:
(1) a linker polypeptide located between a C terminal of the protein A and an N terminal of the protein B, the second amino acid residue from the last at the C terminal of the linker polypeptide and the last amino acid residue at the C terminal being Lys-Arg and the first and second amino acid residues at the N terminal of the linker polypeptide being Arg-Arg, and the linker polypeptide being cut from the fusion protein by intracellular processing in a host; or
(2) a linker polypeptide located between a C terminal of the protein A and an N terminal of the protein B, the second amino acid residue from the last at the C terminal of the linker polypeptide and the last amino acid residue at the C terminal and the first and second amino acid residues at the N terminal of the linker polypeptide being constituted by basic amino acids selected from a group consisting of Arg and Lys, or hydrophobic amino acids selected from a group consisting of Pro, Phe, Leu, Tyr, Ile, Leu and Val.

Particularly preferably, X is formed of 31-36 amino acid residues and has an amino acid sequence indicated as sequence Nos. 1-5. It is possible to modify the amino acid sequences indicated as sequence Nos. 1-5 into other sequences (hereinafter, referred to as "similar amino acid sequences") by substitution of any of the amino acids, addition, deletion or the like. Examples of such a modification method are methods using suitable restriction enzymes, DNA synthetase, DNA ligase, methods known as oligonucleotide directed mutagenesis), methods described by, for example, Maniatis et al. Molecular Cloning 2nd edition (1989) 15.3-109 or Ausubel et al. Current Protocols in Molecular Biology (1991) and the like, methods described in references cited in those publications, and the like.

According to the present invention, there is also developed and provided a bioactive fusion protein characterized by containing an amino acid sequence indicated as sequence Nos. 1-5 or a similar amino acid sequence, or a expression vector for producing the bioactive fusion protein. A fusion protein or a fusion protein expression vector according to the present invention characterized by comprising a DNA sequence encoding a linker polypeptide amino acid sequence or a similar amino acid sequence is not particularly limited as long as it comprises a DNA sequence encoding an amino acid sequence that substantially retains the function and structure.

Examples of a vector suitable for expression of the fusion protein in an Escherichia coli strain may be bacteriophages such as bacteriophage λ or their derivatives, plasmids such as pBR317, pBR322 or pUC vectors, or M13-type phages and their derivatives, and the like. However, the vector is not particularly limited as long as it contains a replication origin and a marker gene in complete forms. The marker gene used herein is not particularly limited as long as it is a gene that enables selection and identification based on phenotypic characters of microorganisms transformed by an expression plasmid and that preferably enables selection of transformants by providing the microorganisms with resistance to heavy metals or antibiotics, for example, ampicillin, tetracycline or the like.

Examples of an expression regulating sequence for regulating the expression cassette in Escherichia coli may be lac or tac promoter, lpp promoter, trc promoter improved from tac promoter, or pL promoter of phage λ. Trp, lac and tac promoters may preferably be used.

As a signal sequence used in Escherichia coli, a gene encoding a normally secreted polypeptide may be selected, for example, ompA, lpp, maltose binding protein, λ receptor or β-lactamase signal sequence, or the like.

A vector for replication and expression in Saccharomyces cerevisiae is not particularly limited as long as it contains a yeast replication origin and a selective gene marker characteristic for yeast. A vector containing a chromosome autoreplication sequence (ARS) or a hybrid vector containing a sequence equivalent to yeast 2 µ plasmid DNA, or ARS and a chromosome centromere sequence, for example, CEN4, may preferably used. The marker gene suitable for yeast is a gene that provides a host with resistance to an antibiotic or, in the case of a nutrient requiring yeast mutant, a gene that supplement a damage to a host, for example, a gene that provides resistance to cycloheximide antibiotic or prepares for prototrophy in the nutrient requiring yeast mutant. Preferably, a hybrid vector containing a replication origin and a maker gene for a bacterial host, particularly, Escherichia coli, which enables production and cloning of a gene encoding a fusion protein in Escherichia coli, may be used.

Examples of an expression regulating sequence suitable for expression in yeast may be CYC1, CAL1/10 or PH05 gene promoter, a promoter involved in glycolysis, α-factor promoter, a hybrid promoter, and the like. Examples of the signal sequence used in yeast may be signal sequences of yeast impeldase or pheromone peptidase. However, the signal sequence is not particularly limited as long as it is known in the field.

As a vector for replication and expression in mammalian cells, DNA derived from viruses, for example, simian virus 40 (SV40), roast papilloma virus (RSV), adenovirus 2, bovine papilloma virus (BPV), papovavirus (BXV), or mouse or human cytomegalovirus (each MCMV and HCMV), may be used. Preferred vectors is a hybrid vector containing a replication origin and a maker gene for a bacterial host, particularly, Escherichia coli, which enables production and cloning of a gene encoding a fusion protein in Escherichia coil.

Examples of an expression regulating sequence or a promoter suitable for use in mammalian cells are, in particular, early and late promoters of SV40, a promoter of mouse metallothionein gene, an enhancer-promoter region of mouse or human cytomegalovirus early gene, a human immunoglobulin enhancer-promoter region, a glucocorticoid-inducing promotoer in a mouse papilloma virus long terminal repeat (MMTV LTR), a promoter region of an enhancer-promoter mouse metallothionein gene derived from a long terminal repeat of mouse sarcoma virus, and the like.

As a marker gene for use in mammalian cells, resistant genes well known in the field may be used without limitations, for example, neo or ble gene from a transposon T that provides resistance to an antibiotic G-418 or bleomycin-type antibiotics, Escherichia coli hygromycin B resistance gene, or thymidine kinase gene of the herpes simplex virus that converts a TK- cell strain into TK+ cells, and the like.

The hybrid vector preferably used for mammalian cells may be a hybrid vector carrying a 3' non-translated region of mammalian gene containing signals for proper transcription termination, mRNA splicing site and polyadenylation, for example, a hybrid vector carrying a SV40-derived VP1 processing signal and an antibiotic resistance gene or a replication origin for proliferation in Escherichia coli.

The expression cassette used in the present invention is read in a non-interrupted single frame in which the 5' terminal of a gene encoding a bioactive protein A starts with ATG, and the 3' terminal of a gene encoding a bioactive protein B ends with a translation termination codon. The genes encoding bloactive proteins may be suitably modified partially in their DNA sequences or amino acid sequences in order to link to a linker polypeptide, as long as the genes retain their original functions. The expression cassette A-X-B may be coupled and prepared by methods known in the field utilizing, for example, restriction enzymes and/or synthesized DNA linker molecules and/or blunt end ligation.

Further, a bioactive fusion protein expression vector used in the present invention characterized by containing a DNA sequence encoding an amino acid sequence or similar amino acid sequence is able to produce a large amount of cells termed transformants having foreign genes with a bioactive fusion protein or a capability of producing a bioactive fusion protein by introducing the vector into a suitable host, for example, host cells, in accordance with an ordinary known method, to transform the host cells, and then growing the thus-transformed host cells by a method including culture and the like. Examples of the host to be used may be bacterias such as Escherichia coli, Bacillus subtilis and the like, yeasts such as Saccharomyces cerevisiae, its mutants and the like, established human or animal cell strains such as myeloma cell strains, mouse LTK cell strains, human malignant melanoma Bowes cell strains, HeLa cell trains, COS cell strains, that is, SV40 virus-transformed kidney cell strains of African green monkey CV-1 cells, Chinese hamster ovary (CHO) cell strains, and their sports, and the like. Saccharomyces cerevisiae, Escherichia coli, and eucaryotic cells such as mammalian cells, particularly, COS cells, CHO cells or the like, may preferably be used. It should be understood that further hosts known in the field, such as insect cells represented by silk work, Autographica Californica(baculovirus) and the like, may be used without any limitation.

Transformation of prokaryotic cells or eukaryotic cells may employ methods used in the field. For example, the transformation of Escherichia coli using a plasmid can be accomplished in accordance with a method described by Maniatis et al., Molecular Cloning 2nd edition (1989) 1. 74-84, and the transformation of yeast using a hybrid vector can be accomplished in accordance with a method described by Hinnen et al., Proc. Natl. Acad. Sci. USA 75, 1919 (1978). The transformed host cells may advantageously be isolated from a selection nutrition medium containing an antibiotic or the like to which the marker gene contained in the expression plasmid produces resistance. For example, if a hybrid vector used carries a β-lactamase gene, ampicillin is correspondingly added to the nuetrition medium.

As for a method for introducing an expression vector into a mammalian cells, methods normally used in the field of gene recombination technology may be used. For example, a calcium phosphate co-precipitation method, a diethylaminoethyl dextran (DEAE-Dextran) method, an electric pulse method, a micro-injection method, a virus vector method or the like may suitably be used. The transformed cells may be selected using a selection marker incorporated into the expression vector in a covalent-bonded manner or added separately therefrom. Examples of the selection marker to be used are a gene that provided resistance to the antibiotic used, a gene that supplements a genetic defect of the host cells, and the like.

According to the present invention, there is provided, in particular, an improved technique for gene manipulation using a linker polypeptide, a technique for purification of a fusion protein using an anti-body against a linker polypeptide, a technique for producing a dimer protein having a disulfide bond, a technique for producing a fusion protein having two different functions, or the like.

Transformed host cells are cultured in a liquid medium containing anabolizable carbon source, nitrogen source and inorganic salts by a method known in the field. Escherichia coli, yeast or mammalian cells, insect cells or the like transformed in accordance with the present invention may be grown or culture in various ordinary cell culture media. Examples of the carbon source include, but are not limited to, anoblizable carbohydrates such as glucose, maltose, mannitol, lactose, and the like. Examples of the nitrogen source include, but are not limited to, amino acids, casamino acids, peptides, proteins, and their decomposed substances such as trypton or peptone, meat extract, yeast extract, malt extract, ammonium salts such as ammonium chloride, ammonium sulfate, ammonium nitrate and the like. Examples of the inorganic salts that may be used are sulfuric acid salts, chlorides, phosphoric acid salts, carbonic acid salts and the like of sodium, potassium, magnesium, and calcium. The medium may further contain vitamins, antibiotics, and the like. These ingredients may be suitably used alone or in the form of a mixture. A preferable medium may be a commercially and widely available medium or may be prepared by suitably modifying such a medium.

To grow or culture the transformants, methods used in the field may be used. For example, the conditions suitable for the growth of the transformants, such as pH, temperature, air ventilation, carbon dioxide concentration, frequency of medium replacement, and the like, may be suitably determined based on experiments or the like.

After growth or culture the transformants, a bioactive fusion protein according to the present invention containing a linker polypeptide or a fusion protein formed by removal of the linker polypeptide through intracellular processing in the host cells may be separated and collected by an ordinary procedure.

Example of the separating method are a method utilizing co-precipitation from a culture supernatant with a protein precipitating agent, an extraction method using a buffer containing various salts, a solubilization or precipitation method using an acid or alkali, an extraction or precipitation method using an organic solvent, a salting-out method using a protein precipitating agent such as ammonium sulfate or the like, dialysis, ultra-filtration using a membrane filter, gel filtration chromatography, adsorption chromatography, reverse-phase chromatography, affinity chromatography, high speed liquid chromatography, isoelectric focusing or gel electrophoresis, and the like. These methods or techniques may be used alone or in a suitable combination. Purification of a fusion protein containing a linker polypeptide may be performed by, in addition to the aforementioned methods, affinity chromatography, for example, antibody affinity chromatography, particularly, polyclonal or monoclonal antibody affinity chromatography using an antibody (which recognizes, for example, a linker polypeptide present in the fusion protein) fixed to an insoluble matrix by a method known in the field. This method may used alone or in a suitable combination.

The bioactive fusion protein produced and purified according to the present invention may be applied to a curative medicine or a diagnostic agent, or gene therapy by using a fusion protein expression vector characterized by containing a DNA sequence encoding an amino acid sequence of the fusion protein. For example, since interleukin-12 is a heterodimer composed of two subunits p35 and p40, the two subunits must be simultaneously expressed in order to obtain a physiological activity. Therefore, the conventional methods require that two kinds of expression vectors be incorporated but the expression amounts by the vectors vary. Thus it is difficult to obtain transformants into which the two kinds of vectors have been incorporated in equal amounts. Moreover, the subunit p40 of the interleukin-12 (Japanese Patent Laid-Open No. Hei 6-329549) and the p40 homodimer (Japanese Patent Laid-Open No. Hei 7-53594), which act as antagonist to interleukin-12, are also produced. However, according to the present invention, the heterodimer interleukin-12 can be expressed without allowing expression of the antagonist p40 or p40 homodimer. The fusion protein exhibits biological activities similar to those of interleukin-12, for example, reaction with an anti-IL12gp40 monoclonal antibody, promotion of growth of 2D6 cells, that is, interleukin-12 growth-dependent cells, and the like. Therefore, the present invention will provide a gene therapeutic method using interleukin-12 with an increased efficiency compared with the conventional art. The method of the invention is not limited to interleukin-12, but may also be applied to other hetero-dimer proteins and, further, homodimer proteins.

The present invention achieves the following advantages.

By selecting an optimal linker polypeptide for use according the present invention, it becomes possible to efficiently produce a novel bioactive fusion protein. For example, a novel protein, a dimer protein of interleukin-2, an IL2-X-IL2 fusion protein, that does not naturally occur can also be produced. The produced IL2-X-IL2 fusion protein exhibits biological activities substantially the same as those of interleukin-2, for example, promotion of growth of CTLL-2 cells, that is, interleukin-2 growth-dependent cells, and the like. Therefore, it is expected that the IL12gp40-X-IL12gp40 fusion protein or the IL2-X-IL2 fusion protein can be used in various disease diagnosis and therapy, such as clinical treatment of patients with autoallergic disease such as chronic rheumatism, diabetes and the like, cancer therapy, therapy of virus infections, or diagnosis thereof, and the like.

### EXAMPLES

The present invention will be described further in detail with reference to examples. The examples are merely for illustration of the invention and do not limit the invention defined in the claims in any way.

### EXAMPLE 1 ISOLATION OF LINKER POLYPEPTIDE (1)

Use of insulin C chain as a linker polypeptide was examined. First, total RNA was prepared from mouse pancreas in accordance with a method described in ISOGEN (Nippon Gene Kabushiki Gaisha). Using 1 µg of the total RNA as a template and oligo(dT)₁₅ as a primer, 1st strand cDNA was synthesized in accordance with a procedure described in SuperScript Preamplification System (Life Technologies Inc.). Using 1/20 amounts of the synthesized 1st strand cDNA as a template and 20 pmol of each of the primers specific to the insulin C chain indicated as Sequence Nos. 6 and 7, PCR was performed (reaction conditions: 32 cycles of 94°C, 40sec -> 63°C, 40sec -> 72°C, 1 min. reaction mixture composition: 2.5 units of Ex Taq (Takara Shuzo Kabushiki Gaisha), 1x Ex Taq Buffer, 0.2 mM dNTP, total 50 µL), thereby simultaneously amplifying insulin type I C chain DNA fragments of about 100 base pairs and insulin type II C chain DNA fragments of about 110 base pairs. Through agarose gel electrophoresis, DNA fragments were isolated and purified. After blunting and phosphorylation using a commercially available kit (SureClone, Pharmacia Kabushiki Gaisha), the DNA fragments were inserted into the cloning vector pUC18 (cut with the SmaI restriction enzyme and treated with alkali phosphatase) of the SureClone kit (Pharmacia Kabushiki Gaisha) using a ligation kit (Takara Shuzo Kabushiki Gaisha). After transformation of competent cell Escherichia coli JM 109 (Takara Shuzo Kabushiki Gaisha), the recombinant was left in an agar medium containing 100 µg/mL of ampicillin, 0.4 mM of IPTG and 40 µg/mL of X-gal at 37°C overnight for selection. Then, ten white colonies were selected and cultured at 37°C for 6 hours followed by preparation of plasmid DNA using an automatic plasmid extractor (PI-100 by Kurashiki Bouseki Kabushiki Gaisha). Then, presence/absence of insert fragments was confirmed by digestion with the PvuII restriction enzyme. With regard to five clones wherein presence of insert fragments was confirmed, the nucleotide sequences were analyzed using ABI 373A DNA Sequencer (Perkin Elmer Kabushiki Gaisha), thereby obtaining clones InsCI/pUC18 and InsII/pUC18 containing insulin type I and type II C chains. The amino acid sequences of the sense strands of the thus-obtained insulin type I and type II C chains are indicated as Sequence Nos. 1 and 2, and the amino acid sequences of the anti-sense strands thereof are indicated as Sequence Nos. 3 and 4.

### EXAMPLE 2 ISOLATION OF LINKER POLYPEPTIDE (1) (2)

To isolate from the chromosome DNA of Escherichia coli DNA fragments that could be linker polypeptides, chromosome DNA was prepared from Escherichia coli JM 109 by a cesium chloride density gradient centrifugation method (Maniatis et al., Molecular Cloning 2nd edition (1989) 1.42-1.52). 2 µg of the DNA was then reacted with 20 units of the StuI restriction enzyme at 37°C for 2 hours. Through agarose gel electrophoresis, DNA fragments of about 100 base pairs were isolated and purified.

A StuI restriction enzyme site was introduced between the BamHI and XbaI restriction enzyme sites of the cloning vector pUV18 to prepare a new vector pUC18(StuI), which was cut by the StuI restriction enzyme and dephosphorylated by alkali phosphatase. Using the ligation kit (Takara Shuzo Kabushiki Gaisha), the DNA fragments were inserted into the pUC18(StuI). After transformation of competent cell Escherichia coli JM 109 (Takara Shuzo Kabushiki Gaisha), the recombinant was left in an agar medium containing 100 µg/mL of ampicillin, 0.4 mM of IPTG and 40 µg/mL of X-gal at 37°C overnight for selection. Then, ten white colonies were selected and cultured at 37°C for 6 hours followed by preparation of plasmid DNA using an automatic plasmid extractor (PI-100 by Kurashiki Bouseki Kabushiki Gaisha). Then, presence/absence of insert fragments was confirmed by incision with the PvuII restriction enzyme. With regard to eight clones wherein presence of insert fragments was confirmed, the nucleotide sequences were analyzed using ABI 373A DNA Sequencer (Perkin Elmer Kabushiki Gaisha), thereby finding clone having an insert DNA fragment having a conforming reading frame during use as a linker polypeptide and having no termination codon, that is, E. coli/pUC18. The amino acid sequence originating from the thus-obtained Escherichia coli genome DNA is indicated as Sequence No. 5.

### EXAMPLE 3 CONSTRUCTION OF IL12gp40-X-IL12gp35 (1)

To produce a fusion protein having a linker polypeptide X, a dimer protein IL12 composed of a gp40 subunit and a gp35 subunit was first used. In accordance with a normal method, mouse-derived splenic cells (about 8 x 10⁶ cells) were obtained. The cells were culture in a 10% FCS-containing RPMI1640 containing 1% pork weed mitogen at 37°C in the presence of 5% carbon dioxide for 24 hours. After collection of cells through centrifugation, total RNA was prepared in accordance with the method described in ISOGEN (Nippon Gene Kabushiki Gaisha). Using 1 µg of the total RNA as a template and oligo(dT)₁₅ as a primer, 1st strand cDNA was synthesized in accordance with the procedure described in SuperScript Preamplification System (Life Technologies Inc.). Using 1/20 amounts of the synthesized 1st strand cDNA as a template and 20 pmol of each of the primers specific to the gp40 and gp35 indicated as Sequence Nos. 8, 9 and 10, 11, PCR was performed (reaction conditions: 32 cycles of 94°C, 1min -> 63°C, 2min -> 72°C, 4 min. reaction mixture composition: 2.5 units of Ex Taq (Takara Shuzo kabushiki Gaisha), 1x Ex Taq Buffer, 0.2 mM dNTP, total 50 µL), thereby simultaneously amplifying pg40 cDNA fragments and gp35 cDNA fragments not containing a gp35 signal sequence. Blunting and phosphorylation were performed using a commercially available kit (SureClone, Pharmacia Kabushiki Gaisha). After being isolated and purified through agarose gel electrophoresis, the DNA fragments were inserted into the cloning vector pUC18 (cut with the SmaI restriction enzyme and treated with alkali phosphatase) of the SureClone kit (Pharmacia Kabushiki Gaisha) using a ligation kit (Takara Shuzo Kabushiki Gaisha). After transformation of competent cell Escherichia coli JM 109 (Takara Shuzo Kabushiki Gaisha), the recombinant was left in an agar medium containing 100 µg/mL of ampicillin, 0.4 mM of IPTG and 40 µg/mL of X-gal at 37°C overnight for selection. Then, ten white colonies were selected from each clone and cultured at 37°C for 6 hours followed by preparation of plasmid DNA using an automatic plasmid extractor (PI-100 by Kurashiki Bouseki Kabushiki Gaisha). The gp40 clone was digested with the PvuII restriction enzyme, and the gp35 clone was digest with the XbaI restriction enzyme. The size and direction of the insert fragments were analyzed by agarose gel electrophoresis. With respect to three of each clone having cDNA fragments inserted in a suitable direction, the nucleotide sequences were analyzed using ABI 373A DNA Sequencer (Perkin Elmer Kabushiki Gaisha), thereby determining clones (gp40+S/pUC18 and gp35-S/pUC18) having a target cDNA fragment and having no mutation (Fig. 1).

The gp35-S/pUC18 clone was digested with the EcoRI restriction enzyme, and blunted and phosphorylated using a commercially available kit (Pharmacia Kabushiki Gaisha), and then cut with the HindIII restriction enzyme. Through agarose gel electrophoresis, DNA fragment (about 800 base pair) was isolated and purified. The gp40+S/pUC18 was cut with the HindII and HindIII restriction enzymes. After dephosphorylation of the gp40+S/pUC18 by alkali phosphatase, the gp35-S fragment was inserted into the gp40+S/pUC18 vector using the ligation kit (Takara Shuzo Kabushiki Gaisha). After transformation of competent cell Escherichia coli JM 109 (Takara Shuzo Kabushiki Gaisha), the recombinant was left in an agar medium containing 100 µg/mL of ampicillin at 37°C overnight for selection. Eight colonies obtained were cultured at 37°C for 6 hours followed by preparation of plasmid DNA using an automatic plasmid extractor (PI-100 by Kurashiki Bouseki Kabushiki Gaisha). Then, presence/absence of insert fragments was confirmed by incision with the BamHI restriction enzyme, thereby producing gp40-gp35 clone. Fig. 1 illustrates the overall experiment procedure.

### EXAMPLE 4 CONSTRUCTION OF IL12gp40-X-IL12gp35 (2)

The two types of clones obtained in Example 1 were cut with the StuI and SnaBI restriction enzymes. Through agarose gel electrophoresis, DNA fragments of about 100 base pairs (insulin type I C chain) and about 110 base pairs (insulin type II C chain) were isolated and purified.

The gp40-gp35 clone obtained in Example 3 was digested with the SnaBI and SmaI restriction enzymes, and dephosphorylated by alkali phosphatase. After insertion of the aforementioned DNA fragments using the ligation kit (Takara Shuzo Kabushiki Gaisha), competent cell Escherichia coli JM109 (Takara Shuzo Kabushiki Gaisha) was transformed. The recombinant was left in an agar medium containing 100 µg/mL of ampicillin at 37°C overnight for selection. Then, ten colonies were selected and cultured at 37°C for six hours followed by preparation of plasmid DNA using an automatic plasmid extractor (PI-100 by Kurashiki Bouseki Kabushiki Gaisha). Presence/absence of insert fragments was confirmed by digestion with the BamHI restriction enzyme, and the nucleotide sequence was analyzed using ABI 373A DNA Sequencer (Perkin Elmer Kabushiki Gaisha), thereby confirming clones (InsCI-C, N/pUC18; InsCII-C, N/pUC18) containing inserts of insulin type I C chain and type II C chain in forward or reverse direction. Figs. 2-9 illustrate the experiment procedures. In short, InsI-N/pUC18, InsCII-N/pUC18 containing as linker polypeptides the amino acid sequences of the sense strands of insulin type I C chain and type II C chain indicated as Sequence Nos. 1, 2, and InsI-C/pUC18, InsCII-C/pUC18 containing as linker polypeptides the amino acid sequences of the anti-sense strands of insulin type I C chain and type II C chain indicated as Sequence Nos. 3, 4 were constructed.

### EXAMPLE 5 CONSTRUCTION OF IL12gp40-X-IL12gp35 (3)

The clone obtained in Example 2 was digested with the StuI restriction enzyme. Through agarose gel electrophoresis, DNA fragments of about 100 base pairs were isolated and purified.

The gp40-gp35 clone obtained in Example 3 was cut with the SnaBI and SmaI restriction enzymes, and dephosphorylated by alkali phosphatase. After insertion of the aforementioned DNA fragments using the ligation kit (Takara Shuzo Kabushiki Gaisha), competent cell Escherichia coli JM109 (Takara Shuzo Kabushiki Gaisha) was transformed. The recombinant was left in an agar medium containing 100 µg/mL of ampicillin at 37°C overnight for selection. Then, ten colonies were selected and cultured at 37°C for six hours followed by preparation of plasmid DNA using an automatic plasmid extractor (PI-100 by Kurashiki Bouseki Kabushiki Gaisha). Presence/absence of insert fragments was confirmed by digestion with the BamHI restriction enzyme, and the nucleotide sequence was analyzed using ABI 373A DNA Sequencer (Perkin Elmer Kabushiki Gaisha), thereby confirming a clone (E. coli-N/pUC18) containing E. coli genome as a linker polypeptide. Figs. 10 and 11 illustrate the experiment procedures. In short, E. coli-N/pUC18 containing as a linker polypeptide the amino acid sequence originating from the Escherichia coli genome indicated as Sequence No. 5 was constructed.

### EXAMPLE 6 EXPRESSION OF IL12gp40-X-IL12gp35 (1)

Five types of clones constructed in Examples 4 and 5 were cut by the BamHI restriction enzyme. Through agarose gel electrophoresis, DNA fragments of about 1.9 kilo base pairs from the individual clones were isolated and purified. Similarly, an eukaryotic cell expression vector pSVL (Pharmacia Kabushiki Gaisha) was cut with the BamHI restriction enzyme, and dephosphorylation by alkali phosphatase was performed. The aforementioned DNA fragments were inserted into the vector using a ligation kit (Takara Shuzo Kabushiki Gaisha) before transformation of competent cell Escherichia coli JM 109 (Takara Shuzo Kabushiki Gaisha). The recombinant was left in an agar medium containing 100 µg/mL of ampicillin at 37°C overnight for selection. Then, ten colonies were selected for each DNA fragment type and cultured at 37°C for 6 hours followed by preparation of plasmid DNA using an automatic plasmid extractor (PI-100 by Kurashiki Bouseki Kabushiki Gaisha). Then, presence/absence and direction of insert fragments were confirmed by digestion with the SacI restriction enzyme, thereby obtaining InsCI-C, N/pSVL and InsCII-C, N/pSVL and E. coli-N/pSVL. The nucleotide sequences of joint regions between gp40 and InsC and between InsC and gp35 were analyzed using ABI 373A DNA Sequencer (Perkin Elmer Kabushiki Gaisha), thereby confirming that a fusion protein was producible from a single reading frame (Figs. 2-11).

Using 1/20 amounts of the 1st strand cDNA synthesized in Example 3 as a template and using 20 pmol of each of the primers specific to the gp40 indicated as Sequence Nos. 8 and 12 and to the gp35 indicated as Sequence Nos. 11 and 13, PCR was performed (reaction conditions: 32 cycles of 94°C, 1min -> 54°C, 2min -> 72°C, 4 min. reaction mixture composition: 2.5 units of Ex Taq (Takara Shuzo kabushiki Gaisha), 1x Ex Taq Buffer, 0.2 mM dNTP, total 50 µL), to amplify the gp40 and gp35 cDNA fragments containing signal sequence regions. After blunting and phosphorylation using a commercially available kit (SureClone, Pharmacia Kabushiki Gaisha), agarose gel electrophoresis was performed for isolation and purification of the DNA fragments. The DNA fragments were then inserted into the cloning vector pUC18 (digested with the SmaI restriction enzyme and treated with alkali phosphatase) of the SureClone kit (Pharmacia Kabushiki Gaisha) using a ligation kit (Takara Shuzo Kabushiki Gaisha). After transformation of competent cell Escherichia coli JM 109 (Takara Shuzo Kabushiki Gaisha), the recombinant was left in an agar medium containing 100 µg/mL of ampicillin, 0.4 mM of IPTG and 40 µg/mL of X-gal at 37°C overnight for selection. Then, ten white colonies for each were selected and cultured at 37°C for 6 hours followed by preparation of plasmid DNA using an automatic plasmid extractor (PI-100 by Kurashiki Bouseki Kabushiki Gaisha). The size and direction of inserts fragments were analyzed by digestion of the gp40 clones with the PvuII restriction enzyme and the gp35 clones with the XbaI restriction enzyme followed by agarose gel electrophoresis. With respect to three clones contaning insert cDNA fragments in suitable directions, the nucleotide sequences were analyzed using ABI 373A DNA Sequencer (Perkin Elmer Kabushiki Gaisha), thereby determining clones (gp40/pUC18 and gp35/pUC18) containing the desired cDNA fragments and having no mutation. The obtained two clones were digested with the BamHI restriction enzyme, and DNA fragments of about 800 base pairs were isolated and purified from each clone through agarose gel electrophoresis. Similarly, an eukaryotic cell expression vector pSVL (Pharmacia Kabushiki Gaisha) was cut with the BamHI restriction enzyme, and dephosphorylation by alkali phosphatase was performed. The aforementioned DNA fragments were inserted into the vector using a ligation kit (Takara Shuzo kabushiki Gaisha) before transformation of competent cell Escherichia coli JM 109 (Takara Shuzo Kabushiki Gaisha). The recombinant was left in an agar medium containing 100 µg/mL of ampicillin at 37°C overnight for selection. Then, ten colonies were selected for each DNA fragment type and cultured at 37°C for 6 hours followed by preparation of plasmid DNA using an automatic plasmid extractor (PI-100 by Kurashiki Bouseki Kabushiki Gaisha). Then, presence/absence and direction of insert fragments were confirmed by digestion with the XbaI restriction enzyme, thereby obtaining gp35/pSVI and gp40/pSVL.

The thus-constructed seven types of cloned DNA were prepared in large amounts by an alkali-SDS method, and then used as mammalian cell transforming DNA.

### EXAMPLE 7 EXPRESSION OF IL12gp40-X-IL12gp35 (2)

Using the DNA prepared in Example 6 and pSVL as a control, the aforementioned COS cells were transformed by a DEAE-Dextran method (Maniatis et al., Molecular Cloning 2nd edition (1989) 16.41-46; Sharp et al., Current Protocols in Molecular Biology (1991) 16.13; ARAI Naoko, Genetic Engineering Handbook (1992) 194-198). In accordance with a normal method, a solution obtained by mixing 4.45 mL of a RPMI-2%FCS-50µM β-mercaptoethanol-kanamycin solution, 0.25 mL of 1M Tris-Cl, 0.2 mL of 20 mg/mL DEAE-Dextran (Pharmacia Kabushiki Gaisha), 10 µg of DNA and 10 µL of 100 mM chloroxine, was added to sub-confluent COS cells (about 1 x 10⁸ cells) on 90 mm-plates. After culture at 37°C in the presence of 5% carbon dioxide for 4 hours, the solution was removed and the cells were washed twice with 10 mL of PBS(-). After 20mL of a non-serum medium DMEM/F-12-0.5% NS (Boehringer Mannheim)-kanamycin was added, the cells were cultured at 37°C in the presence of 5% carbon dioxide for 6 days. Subsequent to the culture, each cell supernatant was collected and subjected to filter-sterilization through 0.22 µm filters. The thus-obtained cell supernatants were used as samples for bioassay.

### EXAMPLE 8 EXPRESSION OF IL12gp40-X-IL12gp35 (3)

Using the samples obtained in Example 7, 2D6 assay was performed. 100 µL of the serial dilutions of the samples obtained in Example 7 and 2D6 cells (5 x 103 cells/100 µl/well), which exhibit growth quantitatively dependent on IL12, were placed in each well of 96-well microplates, and then cultured at 37°C in the presence of 5% carbon dioxide for 36 hours. [3H]-Thymidine (18.5 kBq/well) was added 8 hours before the end of culture in order to measure and detect the amount of radio activity taken into the cells as an index of the growth reaction. As the diluent and culture solution, 10% FCS-containing RPMI 1640 was used (Fig. 12).

Results showed that the cell supernatant (InsCI-C/pSVL and InsCII-C/pSVL) wherein fusion proteins having reversely inserted insulin C chains were expressed, exhibited growth activities 60-70 times as much as that of the cell supernatant obtained by a co-transfection method using gp35/pSVL and gp40/pSVL. Similarly, the cell supernatant (InsCI-N/pSVL, InsCII-N/pSVL and E. coli-N/pSVL) wherein fusion proteins having insulin C chain inserts in the forward direction and fusion proteins having Escherichia coli DNA inserts were expressed, exhibited growth activities 30-40 times and 20 times, respectively, as great as that of the cell supernatants obtained by a co-transfection method.

### EXAMPLE 9 EXPRESSION OF IL12gp40-X-IL12gp35 (4)

SDS polyacrylamide gel electrophoresis of the samples obtained in Example 7 was performed under reducing and non-reducing conditions, followed by western blotting with a rat anti-gp40 monoclonal antibody (Fig. 13 and 14). The culture solution obtained in Example 7 was concentrated ten times by centrifugation-filtration using Centricon-30 (Amicon), and then denatured at 95°C for 5 minutes by adding an equal amount of 2x sample buffer (0.125M Tris-Cl, 40% SDS, 0.02% Bromophenol Blue). After each sample was subjected to 10% polyacrylamide gel electrophoresis under non-reducing conditions (without 6%v/v β-mercaptoethanol in the electrophoresis buffer) and reducing (with 6%v/v β- mercaptoethanol added in the electrophoresis buffer), blotting to membranes was performed followed by reaction with rat anti-gp40 monoclonal antibody (supplied from Wistar Institute) and then with mouse anti-rat Ig Cκ chain. Then, 2,000-fold diluted Protein A-HRP (Amersham) was added for color development, thereby detecting bands in the vicinity of 80 kDa and 40-50 kDa in both the fusion proteins having reversely or forwardly-directed inserts of insulin C chains (InsCI-N/pSVL, InsCII-N/pSVL or InsCI-C/pSVL, InsCII-C/pSVL) and the fusion proteins having Escherichia coli DNA inserts (E. coli-N-pSVL) under the non-reducing conditions. However, in the fusion proteins having forwardly-directed inserts of insulin C chains (InsCI-N/pSVL, InsCII-N/pSVL), the bands shifted to 40-50 kDa under the reducing conditions, indicating that these fusion proteins did not have a linker polypeptide according to the present invention but that a heterodimer of gp35 and gp40 was formed by disulfide bonds between the subunits. On the other hand, in the fusion proteins having reverse inserts of insulin C chains (InsCI-C/pSVL, InsCII-C/pSVL, and E. coli-N-pSVL), the band positions did not change in the reducing conditions, indicating that the two subunits of the fusion proteins were joined by linker polypeptides (Fig. 14).

### EXAMPLE 10 CONSTRUCTION OF IL2-X-IL2 (1)

To produce a fusion protein employing a linker polypeptide X, production of a fusion protein of interleukin-2, which acts in a monomer, was examined. After Jurkat cells, that is, human-derived T cells, were stimulated with concanavalin A (Taniguchi et al., Nature 302, p305 (1983)), total RNA was prepared in accordance with a method described in ISOGEN (Nippon Gene Kabushiki Gaisha). Using 1 µg of the total RNA as a template and oligo(dT)₁₅ as a primer, 1st strand cDNA was synthesized in accordance with a procedure described in SuperScript Preamplification System (Life Technologies Inc.). Using 1/20 amounts of the synthesized 1st strand cDNA as a template and 20 pmol of each of the primers specific to the interleukin-2 indicated as Sequence Nos. 14-17, PCR was performed (reaction conditions: 32 cycles of 94°C, 30sec -> 55°C, 1min -> 72°C, 2 min. reaction mixture composition: 2.5 units of Ex Taq (Takara Shuzo Kabushiki Gaisha), 1x Ex Taq Buffer, 0.2 mM dNTP, total 50 µL), thereby amplifying cDNA fragments containing interleukin-2 signal sequence regions and cDNA containing no such signal sequence region. Blunting and phosphorylation were performed using a commercially available kit (SureClone, Pharmacia Kabushiki Gaisha). After isolation and purification through agarose gel electrophoresis, the DNA fragments were inserted into the cloning vector pUC18 (digested with the SmaI restriction enzyme and treated with alkali phosphatase) of the SureClone kit (Pharmacia Kabushiki Gaisha) using a ligation kit (Takara Shuzo Kabushiki Gaisha). After transformation of competent cell Escherichia coli JM 109 (Takara Shuzo Kabushiki Gaisha), the recombinant was left in an agar medium containing 100 µg/mL of ampicillin, 0.4 mM of IPTG and 40 µg/mL of X-gal at 37°C overnight for selection. Then, ten white colonies for each were selected and cultured at 37°C for 6 hours followed by preparation of plasmid DNA using an automatic plasmid extractor (PI-100 by Kurashiki Bouseki Kabushiki Gaisha). Then, presence/absence of insert fragments was confirmed by digestion with the PvuII restriction enzyme followed by agarose gel electrophoresis. With regard to four clones of each type having desired insert fragments, the nucleotide sequences were analyzed using ABI 373A DNA Sequencer (Perkin Elmer Kabushiki Gaisha), thereby determining clones (IL2+S/pUC18 and IL2-S/pUC18) containing desired cDNA insert fragments and no mutation (Fig. 15).

### EXAMPLE 11 CONSTRUCTION OF IL2-X-IL2 (2)

The two types of clones obtained in Example 10 were cut with the StuI and SnaBI restriction enzymes. Through agarose gel electrophoresis, DNA fragments of about 100 base pairs (insulin type I C chain) and about 110 base pairs (insulin type II C chain) were isolated and purified.

The IL2-S/pUC18 clone obtained in Example 10 was digested with the FspI restriction enzyme, and dephosphorylated by alkali phosphatase. After digestion with the EcoRI restriction enzymes, cDNA fragments of about 450 base pairs were isolated and purified through agarose gel electrophoresis. Likewise, the IL2+S/pUC18 was cut with the SmaI and EcoRI restriction enzymes, and then dephosphorylated by alkali phosphatase. Then, the two types of DNA fragments (insulin type I C chain DNA fragments and IL-2 DNA fragments, or insulin type II C chain DNA fragments and IL-2 DNA fragments) were simultaneously inserted using a ligation kit (Takara Shuzo Kabushiki Gaisha) for transformation of competent cell Escherichia coli JM109 (Takara Shuzo Kabushiki Gaisha). The recombinant was then left in an agar medium containing 100 µg/mL of ampicillin at 37°C overnight for selection. Then, ten colonies were selected and combined with primers of IL2-S1 indicated as Sequence No. 14 and InsC-S indicated as Sequence No. 6, and primers of IL2-S1 indicated as Sequence No. 14 and InsC-AS indicated as Sequence No. 7, to directly perform PCR (reaction conditions: 32 cycles of 94°C, 40sec -> 63°C, 40sec -> 72°C, 1min. reaction mixture composition: 2.5 units of Ex Taq (Takara Shuzo kabushiki Gaisha), 1x Ex Taq Buffer, 0.2 mM dNTP, total 50 µL). The reaction products were analyzed by 2% agarose gel electrophoresis, thereby obtaining clones IL2/InsCI-C, N. pUC18 and IL2/InsCII-C, N. pUC18 having desired fragments (Figs. 16-23). In short, IL2/Ins I-N/pUC18, IL2/InsCII-N/pUC18 containing as linker polypeptides the amino acid sequences of the sense strands of insulin type I C chain and type II C chain indicated as Sequence Nos. 1, 2, and IL2/Ins I-C/pUC18, IL2/InsCII-C/pUC18 containing as linker polypeptides the amino acid sequences of the anti-sense strands of insulin type I C chain and type II C chain indicated as Sequence Nos. 3, 4 were constructed.

### EXAMPLE 12 CONSTRUCTION OF IL2-X-IL2 (3)

The four type of clones (IL2/InsCI-C, N/pUC18, IL2/InsCII-C, N/pUC18) constructed in Example 11 were cut with the PstI and KpnI restriction enzymes, and DNA fragments formed of IL2-X-IL2 (about 1 kilo base pairs) were isolated and purified through agarose gel electrophoresis. Similarly, an eukaryotic cell expression vector pSRα (Takabe et al., Mol. Cell Biol., 8, 466 (1988)) was digested with the PstI and kpnI restriction enzymes. After dephosphorylation by alkali phosphatase, the aforementioned DNA fragments were inserted using a ligation kit (Takara Shuzo Kabushiki Gaisha) for transformation of competent cell Escherichia coli JM109 (Takara Shuzo Kabushiki Gaisha). The recombinant was then left in an agar medium containing 100 µg/mL of ampicillin at 37°C overnight for selection. Then, five colonies for each DNA fragment type were selected and cultured at 37°C for 6 hours followed by preparation of plasmid DNA using an automatic plasmid extractor (PI-100 by Kurashiki Bouseki Kabushiki Gaisha). Presence/absence of insert fragments was confirmed by digestion with the XbaI restriction enzyme, thereby obtaining IL2/InsCI-C, N/pSRα, IL2/InsCII-C, N/pSRα. The nucleotide sequences of junction regions between ILS+S and InsC and between InsC and IL2-S were analyzed using using ABI 373A DNA Sequencer (Perkin Elmer Kabushiki Gaisha), thereby confirming that fusion proteins were producible from single reading frames (Figs. 16-23).

Using as 1/20 amounts of the 1st strand cDNA synthesized in Example 10 as a template and 20 pmol of each of the primers specific to the interleukin-2 indicated as Sequence Nos. 14 and 17, PCR was performed (reaction conditions: 32 cycles of 94°C, 30sec -> 55°C, 1min -> 72°C, 2min. reaction mixture composition: 2.5 units of Ex Taq (Takara Shuzo Kabushiki Gaisha), 1x Ex Taq Buffer, 0.2 mM dNTP, total 50 µL), thereby amplifying cDNA fragments containing interleukin-2 signal sequence regions. Blunting and phosphorylation were performed using a commercially available kit (SureClone, Pharmacia Kabushiki Gaisha). After being isolated and purified through agarose gel electrophoresis, the DNA fragments were inserted into the cloning vector pUC18 (digested with the SmaI restriction enzyme and treated with alkali phosphatase) of the SureClone kit (Pharmacia Kabushiki Gaisha) using a ligation kit (Takara Shuzo Kabushiki Gaisha). After transformation of competent cell Escherichia coli JM 109 (Takara Shuzo Kabushiki Gaisha), the recombinant was left in an agar medium containing 100 µg/mL of ampicillin, 0.4 mM of IPTG and 40 µg/mL of X-gal at 37°C overnight for selection. Then, ten white colonies were selected and cultured at 37°C for 6 hours followed by preparation of plasmid DNA using an automatic plasmid extractor (PI-100 by Kurashiki Bouseki Kabushiki Gaisha). Then, presence/absence of insert fragments was confirmed by digestion with the PvuII restriction enzyme followed by agarose gel electrophoresis. With regard to four clones having the desired insert fragments, the nucleotide sequences were analyzed using ABI 373A DNA Sequencer (Perkin Elmer Kabushiki Gaisha), thereby determining clones (IL2/pUC18) containing the desired cDNA insert fragments and no mutation. The obtained clones were cut with the PstI and KpnI, and DNA fragments (about 450 base pairs) were isolated and purified through agarose gel electrophoresis. Using the ligation kit (Takara Shuzo Kabushiki Gaisha), the DNA fragments were inserted into pSRα that has been digested with the PstI and KpnI restriction enzymes and dephosphorylated by alkali phosphatase (Takabe et a., Mol. Cell Biol., 8, 466(1988)) for transformation of competent cell Escherichia coli JM 109 (Takara Shuzo Kabushiki Gaisha). The recombinant was then left in an agar medium containing 100 µg/mL of ampicillin at 37°C overnight for selection. Then, five colonies were selected and cultured at 37°C for 6 hours followed by preparation of plasmid DNA using an automatic plasmid extractor (PI-100 by Kurashiki Bouseki Kabushiki Gaisha). Presence/absence of insert fragments was confirmed by digestion with the XbaI restriction enzyme, thereby obtaining IL2/pSRα.

Large amounts of DNA were prepared from the thus-obtained five types of clones by an alkali-SDS method (Maniatis et al., Molecular Cloning 2nd edition (1989) 1.38-39), and used as mammalian cell transforming DNA.

### EXAMPLE 13 EXPRESSION OF IL2-X-IL2 (2)

Using the DNA prepared in Example 12 and pSRα as a control, COS cells were transformed by a DEAE-Dextran method (Maniatis et al., Molecular Cloning 2nd edition (1989) 16.41-46; Sharp et al., Current Protocols in Molecular Biology (1991) 16.13; ARAI Naoko, Genetic Engineering Handbook (1992) 194-198). In accordance with a normal method, a solution obtained by mixing 4.45 mL of a RPMI-2%FCS-50µM β- mercaptoethanol-kanamycin solution, 0.25 mL of 1M Tris-Cl, 0.2 mL of 20 mg/mL DEAE-Dextran (Pharmacia Kabushiki Gaisha), 10 µg of DNA and 10 µL of 100 mM chloroxine, was added to sub-confluent COS cells (about 1 x 10⁸ cells) on 90 mm-plates. After culture at 37°C in the presence of 5% carbon dioxide for 4 hours, the solution was removed and the cells were washed twice with 10 mL of PBS(-). After 20mL of a non-serum medium DMEM/F-12-0.5% NS (Boehringer Mannheim)-kanamycin was added, the cells were cultured at 37°C in the presence of 5% carbon dioxide for 6 days. Subsequent to the culture, each cell supernatant was collected and subjected to filter-sterilization through 0.22 µm filters. The thus-obtained cell supernatants were used as samples for bio-assay.

### EXAMPLE 14 EXPRESSION OF IL2-X-IL2 (3)

The IL2-X-IL2 fusion proteins present in the samples obtained in Example 13 were measured by a biological activity measurement method (bioassay method) (Mosmann et al., Immunolo. Methods, 65, 55(1983), Sugawara et al., IGAKU NO AYUMI(Development of Medical Science), 128.9 and 733(1984), and the like). 50 µL of the serial dilutions of the samples obtained in Example 13 and CTLL-2 cells (1 x 104 cells/50 µl/well), interleukin-2 dependent cll line, were placed in each well of 96-well microplates, and then cultured at 37°C in the presence of 5% carbon dioxide for 72 hours. Then, 20 µL of 3-(4,5 dimethyl thiazol-z-yl)2,5-dephenyl tetrazolium bromide (MTT) was added, and the culture was left overnight at 37°C in the presence of 5% carbon dioxide. Then, 100 µL of 10% sodium dodecyl sulfate (SDS) solution adjusted by 0.01 M hydrochloric acid was added, and the culture was left overnight at 37°C in the presence of 5% carbon dioxide. On the next day, using a microplate reader (Bio Rad, MODEL 3550), dark-blue formazan formed from MTT was measured by absorption at 595nm (from which the background absorption measured at 655 nm was subtracted), for an index of the growth reaction. As the diluent and culture solution, a medium obtained by adding 50 µM β-mercaptoethanol to 10% FCS-containing RPMI 1640 was used (Fig. 24).

### EXAMPLE 15 EXPRESSION OF IL2-X-IL2 (4)

The samples obtained in Example 13 were concentrated ten times, and then subjected to SDS polyacrylamide gel electrophoresis (Figs. 25 and 26). First, the culture solution obtained in Example 13 was concentrated ten times by centrifugation-filtration using Ultra Free-C3LCC (Nippon Millipore Kogyo Kabushiki Gaisha). After 0.1 µL of 14.4 µM β-mercaptoethanol was added to the total volume of 20 µL, denaturalization was performed at 95°C for 5 minutes by adding an equal volume of 2x sample buffer (0.125M Tris-Cl, 40% SDS, 0.02% Bromophenol Blue). Each sample was subjected to electrophoresis (rated voltage of 130V, 2 hours) on 4-20% concentration gradient precast gel (Tefco Kabushiki Gaisha). Proteins were detected by Coomassie staining method. Comparison of results indicated that, as for the fusion proteins (IL2/Ins IC-N/pSRα, IL2/Ins IIC-N/pSRα) having forwardly-inserted insulin C chains and the fusion proteins (IL2/Ins IC-C/pSRα, IL2/Ins IIC-C/pSRα) having reversely-inserted insulin C chains, IL2-X-IL2 fusion proteins containing linker polypeptide therein were produced regardless of the direction of the insulin C chain, unlike the dimer protein interleukin-12 described in Example 9.

### EXAMPLE 16 PRODUCTION OF IL12gp40-X- IL12gp40/pSRα

The five types of clones constructed in Examples 4 and 5 (InsCI-C N/pUC18, InsCII-C, N/pUC18, E. coli-N/pUC18) were digested with the BamHI restriction enzyme, and then blunted by T4 DNA polymerase. Through agarose gel electrophoresis, DNA fragments of about 1.9 kilo base pairs were isolated and purified from each clone. Similarly, an eukaryotic cell expression vector pSRα (Takabe et al., Mol. Cell Biol., 8, 466(1988)) was digested with the PstI and KpnI restriction enzymes, blunted by T4 DNA polymerase, and dephosphorylated by alkali phosphatase. The aforementioned DNA fragments were inserted into the vectors using a ligation kit (Takara Shuzo Kabushiki Gaisha), for transformation of competent cell Escherichia coli JM 109 (Takara Shuzo Kabushiki Gaisha). The recombinant was then left in an agar medium containing 100 µg/mL of ampicillin overnight at 37°C for selection. Then, ten colonies for each DNA fragment type were selected and cultured at 37°C for 6 hours followed by preparation of plasmid DNA using an automatic plasmid extractor (PI-100 by Kurashiki Bouseki Kabushiki Gaisha). Then, presence/absence and direction of insert fragments were confirmed by digestion with the ClaI restriction enzyme, thereby obtaining InsCI-C,N/pSRα, InsCII-C,N/pSRα, E. coli-N/pSRα. The nucleotide sequences of junction regions between gp40 and InsC and between InsC and gp35 were analyzed using ABI 373A DNA Sequencer (Perkin Elmer Kabushiki Gaisha), thereby confirming that fusion proteins were producible from single reading frames (Figs. 27-31).

The two types of clones produced in Example 6 (gp40/pUC18 and gp35/pUC18) were digested with the BamHI restriction enzyme, and then blunted by T4 DNA polymerase. Through agarose gel electrophoresis, DNA fragments of about 800 base pairs were isolated and purified from each clone. Similarly, an eukaryotic cell expression vector pSRα (Takabe et al., Mol. Cell Biol., 8, 466(1988)) was digested with the PstI and KpnI restriction enzymes, blunted by T4 DNA polymerase, and dephosphorylated by alkali phosphatase. The aforementioned DNA fragments were inserted into the vectors using a ligation kit (Takara Shuzo Kabushiki Gaisha), for transformation of competent cell Escherichia coli JM 109 (Takara Shuzo Kabushiki Gaisha). The recombinant was then left in an agar medium containing 100 µg/mL of ampicillin overnight at 37°C for selection. Then, ten colonies for each DNA fragment type were selected and cultured at 37°C for 6 hours followed by preparation of plasmid DNA using an automatic plasmid extractor (PI-100 by Kurashiki Bouseki Kabushiki Gaisha). Then, presence/absence and direction of insert fragments were confirmed by digestion with the XbaI and HindIII restriction enzymes, thereby obtaining gp35/pSRα and gpSRα/p pSRα.

Large amounts DNA of the thus-constructed seven clones were prepared by an alkali-SDS method (Maniatis et al., Molecular Cloning 2nd edition (1989) 1.38-39), and used as mammalian cell transforming DNA.

### EXAMPLE 17 EXPRESSION OF IL12gp40-X- IL12gp40/pSRα

Using the seven types of DNA prepared in Example 16 and pSRα as a control, the aforementioned COS cells were transformed by a DEAE-Dextran method (Maniatis et al., Molecular Cloning 2nd edition (1989) 16.41-46; Sharp et al., Current Protocols in Molecular Biology (1991) 16. 13; ARAI Naoko, Genetic Engineering Handbook (1992) 194-198). In accordance with a normal method, a solution obtained by mixing 4.45 mL of a RPMI-2%FCS-50µM β- mercaptoethanol-kanamycin solution, 0.25 mL of 1M Tris-Cl, 0.2 mL of 20 mg/mL DEAE-Dextran (Pharmacia Kabushiki Gaisha), 10 µg of DNA and 10 µL of 100 mM chloroxine, was added to sub-confluent COS cells (about 1 x 108 cells) on 90 mm-plates. After culture at 37°C in the presence of 5% carbon dioxide for 4 hours, the solution was removed and the cells were washed twice with 10 mL of PBS(-). After 20 mL of a non-serum medium DMEM/F-12-0.5% NS (Boehringer Mannheim)-kanamyc in was added, the cells were cultured at 37°C in the presence of 5% carbon dioxide for 6 days. Subsequent to the culture, each cell supernatant was collected and subjected to filter-sterilization through 0.22 µm filters. The thus-obtained cell supernatants were used as samples for bioassay.

### EXAMPLE 18 COMPARISON IN EXPRESSION AMOUNT AMONG IL12gp40-X-IL12gp40/pSRα AND IL12gp40-X-IL12gp40/pSVL

Using as indexes the amounts of IL12 present in the cell supernatants obtained in Examples 7 and 17, the amounts of expression by the different vectors pSVL and pSRα were compared. 100 µL of the serial dilutions of the samples obtained in Examples 7 and 17 and 2D6 cells (5 x 103 cells/100 µl/well), which exhibit growth quantitatively dependent on IL12, were placed in each well of 96-well microplates, and then cultured at 37°C in the presence of 5% carbon dioxide for 36 hours. [3H]-Thymidine (18.5 kBq/well) was added 8 hours before the end of culture in order to measure and detect the amount of radio activity taken up by the cells as an index of the growth reaction. Likewise, 100 µL of the serial dilutions of IL12 solution of a known concentration and 2D6 cells (5 x 103 cells/100 µl/well) were placed in each well of 96-well microplates, and then cultured at 37°C in the presence of 5% carbon dioxide for 36 hours. [3H]-Thymidine (18.5 kBq/well) was added 8 hours before the end of culture in order to measure and detect the amount of radio activity taken up by the cells as an index of the growth reaction. Based on each growth curve, the concentration of IL12 contained in each cell supernatant was calculated (Fig. 32). As the diluent and culture solution, 10% FCS-containing RPMI 1640 was used.

Results showed that as for the expression of fusion proteins (InsCI-C, InsCII-C) having reversely inserted insulin C chains, the proteins can be prepared by using the expression vector pSRα with yields 6-10 times as high as that achieved by using pSVL. Similarly, as for the fusion proteins (InsCI-N, InsCII-N) having insulin C chain inserts in the forward direction and the fusion proteins (E. coli-N) having Escherichia coli DNA inserts, the proteins can be produced by using the expression vector pSRα with yields about 6-13 times as high as that achieved by using pSVL.

Therefore, it has become clear that the fusion protein according to the present invention can be produced with an increased yield by using the expression vector pSRα.

### INDUSTRIAL APPLICABILITY

The bioactive fusion protein produced and purified by the present invention can applied to therapeutic or diagnostic agents or can be applied to gene therapy using the fusion protein expression vector characterized by containing a DNA sequence encoding an amino acid sequence of the fusion protein.

SEQUENCE TABLE
Sequence No. 1
Length of Sequence: 33
Type of Sequence: Amino acid
Topology: Linear
Type of Sequence: Peptide
Characters
Symbol Representing Characters: Region
Location: 1..33
Other Information: /Note= Linker polypeptide
Sequence
Arg Arg Glu Val Glu Asp Pro Gln Val Glu Gln Leu Glu Leu Gly
10
Gly Ser Pro Gly Asp Leu Gln Thr Leu Ala Leu Glu Val Ala Arg
20 30
Gln Lys Arg

Sequence No. 2
Length of Sequence: 35
Type of Sequence: Amino acid
Topology: Linear
Type of Sequence: Peptide
Characters
Symbol Representing Characters: Region
Location: 1..35

Other Information: /Note= Linker polypeptide
Sequence
Arg Arg Glu Val Glu Asp Pro Gln Val Ala Gln Leu Glu Leu Gly
10
Gly Gly Pro Gly Ala Gly Asp Leu Gln Thr Leu Ala Leu Glu Val
20 30
Ala Arg Gln Lys Arg

Sequence No. 3
Length of Sequence: 34
Type of Sequence: Amino acid
Topology: Linear
Type of Sequence: Peptide
Characters
Symbol Representing Characters: Region
Location: 1..34
Other Information: /Note= Linker polypeptide
Sequence
Pro Phe Ala Ser Ala Gly Gln Pro Pro Thr Pro Arg Ser Glu Gly
10
Pro Arg Gly Phe Leu Pro Ala Pro Val Val Pro Leu Ala Gly Pro
20 30
Pro Leu Leu Tyr

Sequence No. 4
Length of Sequence: 36
Type of Sequence: Amino acid
Topology: Linear
Type of Sequence: Peptide
Characters
Symbol Representing Characters: Region
Location: 1..36
Other Information: /Note= Linker polypeptide
Sequence
Pro Phe Ala Ser Ala Gly Gln Pro Pro Val Pro Arg Ser Glu Gly
10
His Leu Leu Pro Gly Leu His Pro Ala Pro Val Val Pro Leu Ala
20 30
Gly Pro Pro Leu Leu Tyr

Sequence No. 5
Length of Sequence: 31
Type of Sequence: Amino acid
Topology: Linear
Type of Sequence: Peptide
Characters
Symbol Representing Characters: Region
Location: 1..31
Other Information: /Note= Linker polypeptide

Sequence
Pro Tyr Asn Cys Trp Arg Pro Val Gly Arg Ile Arg His Leu CYs
10
Arg Ile Arg Gln Ser Met Pro Asp Ala Thr Leu Ser Arg Leu Ile
20 30
Arg

Sequence No. 6
Length of Sequence: 24
Type of Sequence: Nucleic acid
Number of Strands: Single strand
Topology: Linear
Type of Sequence: Other nucleic acid, Synthetic DNA
Sequence
TTACGTAGAG AAGTGGAGGA CCCG

Sequence No. 7
Length of Sequence: 24
Type of Sequence: Nucleic acid
Number of Strands: Single strand
Topology: Linear
Type of Sequence: Other nucleic acid, Synthetic DNA
Sequence
AGGCCTTCGC TTCTGCCGGG CAAC

Sequence No. 8
Length of Sequence: 24
Type of Sequence: Nucleic acid
Topology: Linear
Type of Sequence: Other nucleic acid, Synthetic DNA
Sequence
TGCGGATCCG ACCAGGCAGC TCGC

Sequence No. 9
Length of Sequence: 23
Type of Sequence: Nucleic acid
Number of Strands: Single strand
Topology: Linear
Type of Sequence: Other nucleic acid, Synthetic DNA
Sequence
ATCCCGGGAT CGGACCCTGC AGG

Sequence No. 10
Length of Sequence: 24
Type of Sequence: Nucleic acid
Number of Strands: Single strand
Topology: Linear
Type of Sequence: Other nucleic acid, Synthetic DNA
Sequence
TACGTAATTC CAGTCTCTGG ACCT

Sequence No. 11
Length of Sequence: 27
Type of Sequence: Nucleic acid
Number of Strands: Single strand
Topology: Linear
Type of Sequence: Other nucleic acid, Synthetic DNA
Sequence
TTCGGATCCT CCTATCTGTG TGAGGAG

Sequence No. 12
Length of Sequence: 24
Type of Sequence: Nucleic acid
Number of Strands: Single strand
Topology: Linear
Type of Sequence: Other nucleic acid, Synthetic DNA
Sequence
CAACGTTGGA TCCTAGAATC GGAC

Sequence No. 13
Length of Sequence: 27
Type of Sequence: Nucleic acid
Number of Strands: Single strand
Topology: Linear
Type of Sequence: Other nucleic acid, Synthetic DNA
Sequence

GAGGGATCCT CCTGGGAAAG TCTGCCG

Sequence No. 14
Length of Sequence: 28
Type of Sequence: Nucleic acid
Number of Strands: Single strand
Topology: Linear
Type of Sequence: Other nucleic acid, Synthetic DNA
Sequence
TCTGCAGTGC AAAGATGTAC AGGATGCA

Sequence No. 15
Length of Sequence: 25
Type of Sequence: Nucleic acid
Number of Strands: Single strand
Topology: Linear
Type of Sequence: Other nucleic acid, Synthetic DNA
Sequence
TTCCCGGGTC AGTGTTGAGA TGATG

Sequence No. 16
Length of Sequence: 24
Type of Sequence: Nucleic acid
Number of Strands: Single strand
Topology: Linear

Type of Sequence: Other nucleic acid, Synthetic DNA
Sequence
AACTGCGCAC CTACTTCAAG TTCT

Sequence No. 17
Length of Sequence: 28
Type of Sequence: Nucleic acid
Number of Strands: Single strand
Topology: Linear
Type of Sequence: Other nucleic acid, Synthetic DNA
Sequence
AGCGGTACCT TATCAAGTCA GTGTTGAG

## Claims

1. A method for producing a bioactive fusion protein characterized by culturing a transformant obtained by transformation with an expression vector containing a DNA sequence encoding an amino acid sequence of a protein represented by the following formula:
A-X-B
(wherein A and B represent respectively subunits of a dimer protein or bicactive monomers in their native form, where A and B may represent the same, and X represents a linker polypeptide).

2. A method for producing a bioactive fusion protein according to claim 1, characterized in that X is:
(1) a linker polypeptide located between a C terminal of the protein A and an N terminal of the protein B, the second amino acid residue from the last at the C terminal of the linker polypeptide and the last amino acid residue at the C terminal being Lys-Arg and the first and second amino acid residues at the N terminal of the linker polypeptide being Arg-Arg, and the linker polypeptide being cut from the fusion protein by intracellular processing in a host; or
(2) a linker polypeptide located between a C terminal of the protein A and an N terminal of the protein B, the second amino acid residue from the last at the C terminal of the linker polypeptide and the last amino acid residue at the C terminal and the first and second amino acid residues at the N terminal of the linker polypeptide being constituted by basic amino acids selected from a group consisting of Arg and Lys, or hydrophobic amino acids selected from a group consisting of Pro, Phe, Leu, Tyr, Ile, Leu and Val.

3. A method for producing a bioactive fusion protein according to claim 1 or 2, characterized in that X is a linker polypeptide having 31-36 amino acid residues.

4. A method for producing a bioactive fusion protein according to any one of claims 1-3, characterized in that X is a linker polypeptide having an amino acid sequence indicated as any of Sequence Nos. 1-5 or an amino acid sequence exhibiting substantially the same function and structure as the amino acid sequence indicated as any of Sequence Nos. 1-5.

5. A method for producing a bioactive fusion protein according to any one of claims 1-4, characterized by using a mammalian cell strain as a host.

6. An interleukin-12 producing method using any one of the methods described in claims 1-5, characterized in that A and B are a subunit 40kDa of interleukin-12 or a subunit 35KDa of interleukin-12.

7. An expression vector characterized by containing the DNA sequence described in claim 1.

8. A transformant obtained by transforming a host cell with the expression vector described in claim 7.

9. A transformant according to claim 8, characterized in that the host cell is of a mammalian cell strain.


SEQUENCE LISTING

(1) GENERAL INFORMATION:

(i) APPLICANT:
(A) NAME: TORAY INDUSTRIES, INC.
(B) STREET: 2-1, Nihonbashi Muromachi 2-chome, Chuo-ku
(C) CITY: Tokyo
(E) COUNTRY: Japan
(F) POSTAL CODE (ZIP): 103

(A) NAME: TORAY RESEARCH CENTER, INC.
(B) STREET: 1-8, Nihonbashi Muromachi 3-chome, Chuo-ku
(C) CITY: Tokyo
(E) COUNTRY: Japan
(F) POSTAL CODE (ZIP): 103

(ii) TITLE OF INVENTION: Method for producing bioactive fusion protein

(iii) NUMBER OF SEQUENCES: 17

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96 942 608.9
(vi) PRIOR APPLICATION DATA:
(A) APPLICATION NUMBER: PCT/JP96/03742
(B) FILING DATE: 20-DEC-1996

(vi) PRIOR APPLICATION DATA:
(A) APPLICATION NUMBER: JP HEI-7-350364
(B) FILING DATE: 22-DEC-1995

(vi) PRIOR APPLICATION DATA:
(A) APPLICATION NUMBER: JP HEI-7-334875
(B) FILING DATE: 22-DEC-1995

(2) INFORMATION FOR SEQ ID NO: 1:

(i) SEQUENCE CHARACTERISTICS:
(A) LENGTH: 33 amino acids
(B) TYPE: amino acid
(D) TOPOLOGY: linear

(ii) MOLECULE TYPE: peptide

(ix) FEATURE:
(A) NAME/KEY: Region
(B) LOCATION:1..33
(D) OTHER INFORMATION:/note= "Linker polypeptide"

(xi) SEQUENCE DESCRIPTION: SEQ ID NO: 1:

Arg Arg Glu Val Glu Asp Pro Gln Val Glu Gln Leu Glu Leu Gly Gly
1               5                   10                  15

Ser Pro Gly Asp Leu Gln Thr Leu Ala Leu Glu Val Ala Arg Gln Lys
            20                  25                  30

Arg

(2) INFORMATION FOR SEQ ID NO: 2:

     (i) SEQUENCE CHARACTERISTICS:
          (A) LENGTH: 35 amino acids
          (B) TYPE: amino acid
          (D) TOPOLOGY: linear

    (ii) MOLECULE TYPE: peptide

    (ix) FEATURE:
          (A) NAME/KEY: Region
          (B) LOCATION:1..35
          (D) OTHER INFORMATION:/note= "Linker polypeptide"

    (xi) SEQUENCE DESCRIPTION: SEQ ID NO: 2:

Arg Arg Glu Val Glu Asp Pro Gln Val Ala Gln Leu Glu Leu Gly Gly
1               5                   10                  15

Gly Pro Gly Ala Gly Asp Leu Gln Thr Leu Ala Leu Glu Val Ala Arg
            20                  25                  30

Gln Lys Arg
        35

(2) INFORMATION FOR SEQ ID NO: 3:

     (i) SEQUENCE CHARACTERISTICS:
          (A) LENGTH: 34 amino acids
          (B) TYPE: amino acid
          (D) TOPOLOGY: linear

    (ii) MOLECULE TYPE: peptide

    (ix) FEATURE:
          (A) NAME/KEY: Region
          (B) LOCATION:1..34
          (D) OTHER INFORMATION:/note= "Linker polypeptide"

    (xi) SEQUENCE DESCRIPTION: SEQ ID NO: 3:

Pro Phe Ala Ser Ala Gly Gln Pro Pro Thr Pro Arg Ser Glu Gly Pro
1               5                   10                  15

Arg Gly Phe Leu Pro Ala Pro Val Val Pro Leu Ala Gly Pro Pro Leu
            20                  25                  30

Leu Tyr

(2) INFORMATION FOR SEQ ID NO: 4:

     (i) SEQUENCE CHARACTERISTICS:
          (A) LENGTH: 36 amino acids

(B) TYPE: amino acid
(D) TOPOLOGY: linear

(ii) MOLECULE TYPE: peptide

(ix) FEATURE:
(A) NAME/KEY: Region
(B) LOCATION:1..36
(D) OTHER INFORMATION:/note= "Linker polypeptide"

(xi) SEQUENCE DESCRIPTION: SEQ ID NO: 4:

Pro Phe Ala Ser Ala Gly Gln Pro Pro Val Pro Arg Ser Glu Gly His
1               5                   10                  15

Leu Leu Pro Gly Leu His Pro Ala Pro Val Val Pro Leu Ala Gly Pro
            20                  25                  30

Pro Leu Leu Tyr
        35

(2) INFORMATION FOR SEQ ID NO: 5:

(i) SEQUENCE CHARACTERISTICS:
(A) LENGTH: 31 amino acids
(B) TYPE: amino acid
(D) TOPOLOGY: linear

(ii) MOLECULE TYPE: peptide

(ix) FEATURE:
(A) NAME/KEY: Region
(B) LOCATION:1..31
(D) OTHER INFORMATION:/note= "Linker polypeptide"

(xi) SEQUENCE DESCRIPTION: SEQ ID NO: 5:

Pro Tyr Asn Cys Trp Arg Pro Val Gly Arg Ile Arg His Leu Cys Arg
1               5                   10                  15

Ile Arg Gln Ser Met Pro Asp Ala Thr Leu Ser Arg Leu Ile Arg
            20                  25                  30

(2) INFORMATION FOR SEQ ID NO: 6:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 6:

TTACGTAGAG AAGTGGAGGA CCCG                                        24

(2) INFORMATION FOR SEQ ID NO: 7:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 7:

AGGCCTTCGC TTCTGCCGGG CAAC 24

(2) INFORMATION FOR SEQ ID NO: 8:

(i) SEQUENCE CHARACTERISTICS:
(A) LENGTH: 24 base pairs
(B) TYPE: nucleic acid
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 8:

TGCGGATCCG ACCAGGCAGC TCGC 24

(2) INFORMATION FOR SEQ ID NO: 9:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 9:

ATCCCGGGAT CGGACCCTGC AGG 23

(2) INFORMATION FOR SEQ ID NO: 10:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 10:

TACGTAATTC CAGTCTCTGG ACCT 24

(2) INFORMATION FOR SEQ ID NO: 11:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 11:

TTCGGATCCT CCTATCTGTG TGAGGAG 27

(2) INFORMATION FOR SEQ ID NO: 12:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 12:

[?]AACGTTGGA TCCTAGAATC GGAC 24

(2) INFORMATION FOR SEQ ID NO: 13:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 13:

GAGGGATCCT CCTGGGAAAG TCTGCCG 27

(2) INFORMATION FOR SEQ ID NO: 14:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 14:

TCTGCAGTGC AAAGATGTAC AGGATGCA 28

(2) INFORMATION FOR SEQ ID NO: 15:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 15:

TTCCCGGGTC AGTGTTGAGA TGATG 25

(2) INFORMATION FOR SEQ ID NO: 16:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 16:

AACTGCGCAC CTACTTCAAG TTCT 24

(2) INFORMATION FOR SEQ ID NO: 17:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 17:
AGCGGTACCT TATCAAGTCA GTGTTGAG 28